# FASCICULE DE BREVET EUROPEEN

(11) **EP 1 205 175 B1**
(45) Date de publication et mention de la délivrance du brevet: **21.06.2006**
(21) Numéro de dépôt: 01402832.8
(22) Date de dépôt: 31.10.2001
(51) Int. Cl.: A61Q 5/06, A61K 8/04, A61K 8/90

(54) **Mousse coiffante longue tenue**
Langhaltiger Haarschaum
Hair mousse with long-lasting effect

(30) Priorité: 07.11.2000 FR 0014232
(43) Date de publication de la demande: 15.05.2002
(60) Demande divisionnaire: 05011677.1
(73) Titulaire: L'ORÉAL, 75008 Paris (FR)
(72) Inventeur: Belli, Emmanuelle, 92600 Asnières (FR)
(74) Mandataire: Casalonga, Axel

(56) Documents cités:
- WO-A-00/39176
- WO-A-00/40628

## Description

La présente invention concerne une mousse de coiffage contenant un type particulier de copolymère acrylique à blocs ramifié, conditionnée dans un dispositif aérosol.

Bien que l'on connaisse dans le domaine du coiffage un très grand nombre de polymères fixants, la plupart d'entre eux présentent un pouvoir fixant limité dans le temps et qui résiste mal à l'humidité.

On a découvert récemment les propriétés de coiffage très intéressantes d'un groupe particulier de copolymères acryliques séquencés (ou à blocs) ramifiés décrits plus en détail ci-dessous.
Ces copolymères, utilisés dans des compositions de coiffage, présentent une combinaison de propriétés physicochimiques et cosmétiques qui en font d'excellents polymères fixants. Ainsi, ces copolymères séquencés s'étalent facilement sur les cheveux, présentent une bonne adhésion aux fibres capillaires, donnent un toucher peu collant, s'éliminent facilement au shampooing et donnent une fixation satisfaisante d'une bonne élasticité, stable dans le temps et qui résiste particulièrement bien à l'humidité.

La demanderesse a découvert que ces polymères se prêtaient parfaitement bien à un conditionnement sous forme de mousse coiffante dans des dispositifs aérosol.
En effet, les mousses de coiffage, qui constituent une formulation généralement très appréciée par les utilisateurs, présentent l'inconvénient de présenter une tenue insuffisante dans le temps.
Or, les nouveaux polymères fixants acryliques à blocs indiqués ci-dessus - lorsqu'ils sont conditionnés sous forme de composition aérosol - donnent des mousses coiffantes ayant des propriétés de fixation satisfaisantes et présentant une très bonne tenue dans le temps. En outre, les propriétés cosmétiques sont d'un bon niveau.

Dans un mode de réalisation, la présente invention concerne une composition sous forme de mousse de coiffage, conditionnée dans un dispositif aérosol, comprenant
- une phase liquide contenant, dans un milieu liquide cosmétiquement acceptable, (a) au moins un polymère filmogène fixant choisi parmi les copolymères à blocs ramifiés comprenant, comme monomères principaux, au moins un acrylate d'alkyle en C₁₋₂₀ et/ou au moins un N-mono- ou N,N-di-(alkyle en C₂₋₁₂)(méth)acrylamide, et de l'acide acrylique et/ou de l'acide méthacrylique, et (b) au moins un autre polymère filmogène fixant différent des copolymères à blocs ramifiés (a), et
- au moins un agent propulseur.

Le polymère filmogène fixant (a) utilisé dans les compositions cosmétiques de la présente invention est un copolymère séquencé (ou à blocs) ramifié ayant une structure constituée de blocs hydrophobes sur lesquels sont fixées, notamment par l'intermédiaire de motifs bifonctionnels, un certain nombre de blocs plus hydrophiles. Ces copolymères présentent au moins deux températures de transition vitreuse.
Ils sont notamment décrits dans la demande de brevet WO 00/40628.

Les copolymères séquencés ramifiés décrits ci-dessus sont proposés par exemple sous les dénominations EX-SDR-26® et EX-SDR-45® par la société GOODRICH.

Ces copolymères présentent la composition suivante :
- de 26 à 36 % en moles d'acide acrylique
- de 27,5 à 30,5 % en moles d'acrylate de *n*-butyle
- de 33,3 à 45,3 % en moles d'acide métacrylique
- de 0,48 à 0,92 % en moles de méthacrylate d'allyle

Les blocs les plus hydrophobes ont un poids moléculaire de 10 000 à 100 000 et les blocs les plus hydrophiles ont un poids moléculaire de 1000 à 100 000 daltons.

Les polymères filmogènes fixants ci-dessus sont utilisés de préférence sous forme anionique, c'est-à-dire sous forme de sel résultant de la neutralisation partielle ou totale des groupes acide (méth)acrylique. L'agent de neutralisation peut être n'importe qu'elle base minérale ou organique physiologiquement acceptable qui n'interfère pas de manière désavantageuse avec le système épaississant. On peut citer à titre d'exemple d'agent de neutralisation préféré le 2-amino-2-méthyl-1-propanol ou l'hydroxyde de sodium.

Le milieu cosmétiquement acceptable est de préférence un milieu aqueux ou hydroalcoolique, et en particulier un milieu aqueux, contenant le ou les polymères fixants séquencés ramifiés à l'état dissous.

La phase liquide contient de préférence entre 0,1 et 10 % en poids, et en particulier entre 0,5 et 5 % en poids de polymère fixant séquencé ramifié, rapporté au poids total de la phase liquide.

Les polymères filmogènes fixants (b) utilisés en association avec le ou les polymères filmogènes fixants (a) décrits ci-dessus sont choisis de préférence parmi les polymères fixants cationiques, anioniques, non ioniques
ou amphotères énumérés ci-après. Le choix de ces polymères est effectué de façon à obtenir une composition coiffante sous forme de mousse.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.
Parmi ces polymères, on peut citer plus particulièrement les polymères cationiques suivants :
(1) les homopolymères ou copolymères dérivés d'esters ou d'amides acryliques ou méthacryliques et comportant des motifs correspondant à au moins une des formules suivantes : 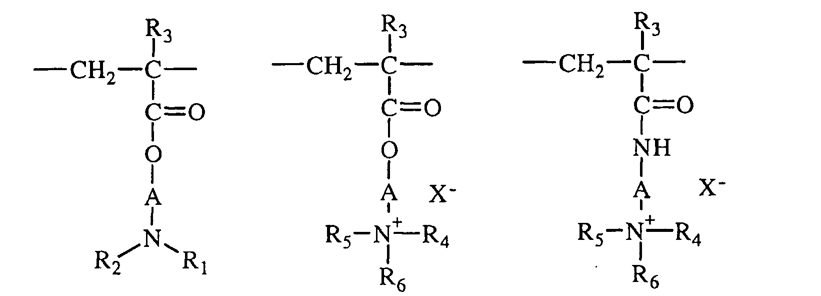 dans lesquelles:
   R₁ et R₂ représentent chacun indépendamment un atome d'hydrogène ou un groupe alkyle en C₁₋₆;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆ représentent indépendamment chacun un groupe alkyle ayant de 1 à 18 atomes de carbone ou un groupe benzyle ;
   X désigne un anion méthosulfate ou halogénure tel que chlorure ou bromure.

   Les copolymères de la famille (1) contiennent en outre un ou plusieurs motifs dérivant de comonomères choisis dans la famille des acrylamides, méthacrylamides, diacétone-acrylamides, acrylamides et méthacrylamides substitués sur l'azote par des groupes alkyle inférieur, des acides (méth)acryliques ou de leurs esters, vinyllactames tels que la vinylpyrrolidone ou le vinylcaprolactame, esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de méthyle tels que celui vendu sous la dénomination HERCOFLOC® par la société HERCULES,
   - les copolymères d'acrylamide et de chlorure de méthacryloyloxy-éthyltriméthylammonium décrits, par exemple, dans la demande de brevet EP-A-080976 et vendus sous la dénomination BINA QUAT® P100 par la société CIBA GEIGY,
   - le copolymère d'acrylamide et de méthosulfate de méthacryloyloxy-éthyltriméthylammonium vendu sous la dénomination RETEN® vendu par la société HERCULES,
   - les copolymères vinylpyrrolidone/acrylate ou méthacrylate de dialkylaminoalkyle quaternisés ou non, tels que les produits vendus sous la dénomination GAFQUAT® par la société ISP comme, par exemple, GAFQUAT® 734 ou GAFQUAT® 755, ou bien les produits dénommés "COPOLYMER® 845, 958 et 937". Ces polymères sont décrits en détail dans les brevets français FR 2 077 143 et FR 2 393 573,
   - les terpolymères méthacrylate de diméthylaminoéthyle/vinylcaprolactame/vinylpyrrolidone tels que le produit commercialisé sous la dénomination GAFFIX® VC 713 par la société ISP, et
   - les copolymères vinylpyrrolidone/méthacrylamide de diméthylaminopropyle quaternisé tels que notamment le produit commercialisé sous la dénomination GAFQUAT® HS 100 par la société ISP.
(2) les polysaccharides quaternisés décrits plus particulièrement dans les brevets américains US 3,589,578 et US 4,031,307 tels que les gommes de guar contenant des groupements cationiques trialkylammonium. De tels produits sont commercialisés notamment sous les dénominations JAGUAR C 13 S, JAGUAR C 15, et JAGUAR C 17 par la société MEYHALL.
(3) les copolymères quaternaires de vinylpyrrolidone et de vinylimidazole, tels que les produits commercialisés par la société BASF sous la dénomination LUVIQUAT TFC.
(4) les chitosanes et leurs sels tels que l'acétate, le lactate, le glutamate, le gluconate ou le pyrrolidone-carboxylate de chitosane.
   Parmi ces composés, on peut citer le chitosane ayant un taux de désacétylation de 90,5 % en poids commercialisé sous la dénomination KYTAN BRUT STANDARD par la société ABER TECHNOLOGIES, le pyrrolidone-carboxylate de chitosane vendu sous la dénomination KYTAMER® PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4,131,576, tels que les hydroxyalkylcelluloses, comme les hydroxyméthylcelluloses, les hydroxyéthylcelluloses et hydroxypropylcelluloses greffées notamment avec un sel de méthacryloyloxyéthyl-triméthylammonium, de méthacrylamidoproyltriméthylammonium ou de diméthyldiallylammonium.
   Ces polymères sont vendus notamment sous les dénominations CELQUAT® L200 et CELQUAT® H100 par la société NATIONAL STARCH.

Les polymères fixants anioniques généralement utilisés sont des polymères comportant des groupements dérivés d'acide carboxylique, d'acide sulfonique ou d'acide phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 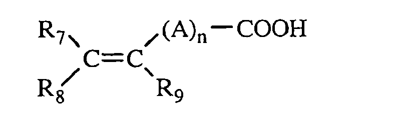 dans laquelle
n est un nombre entier de 0 à 10,
A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre,
R₇ désigne un atome d'hydrogène, un groupement phényle ou benzyle,
R₈ désigne un atome d'hydrogène, un groupement alkyle inférieur ou carboxyle, et
R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL® E ou K par la société ALLIED COLLOID et ULTRAHOLD® par la société BASF, les copolymèrs d'acide acrylique et d'acrylamide vendus sous la forme de sel de sodium sous les dénominations RETEN® 421, 423 ou 425 par la société HERCULES, et les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français FR 1 222 944 et la demande de brevet allemand DE 2 330 956, les copolymères de ce type comportant dans leur chaîne un motif acrylamide éventuellement N-alkylé et/ou N-hydroxyalkylé tels que décrits notamment dans les demandes de brevets luxembourgeois n° 75370 et 75371 et proposés sous la dénomination QUADRAMER® par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés et réticulés, ou encore un autre monomère ester vinylique, allylique ou méthallylique d'un acide carboxylique α- ou β-cyclique. De tels polymères sont décrits entre autres dans les brevets français FR 1 222 944, FR 1 580 545, FR 2 265 782, FR 2 265 781, FR 1 564 110 et FR 2 439 798. Des produits commerciaux entrant dans cette classe sont les résines 28-29-30, 26-13-14 et 28-13-10 commercialisée par la société NATIONAL STARCH.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n° 2,047,398, 2,723,248 et 2,102,113, et le brevet GB 839 805. Des produits commerciaux sont notamment ceux vendus sous les dénominations GANTREZ® AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

   Ces polymères sont par exemple décrits dans les brevets français FR 2 350 384 et FR 2 357 241 de la demanderesse.
E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations FLEXAN® 130 et FLEXAN® 500 par la société NATIONAL STARCH. Ces composés sont décrits dans le brevet FR 2 198 719.
- les sels d'acides polyacrylamide-sulfoniques tels que ceux mentionnés dans le brevet US 4,128,631, et plus particulièrement l'acide polyacrylamidoéthylpropanesulfonique vendu sous la dénomination COSMEDIA POLYMER® HSP 1180 par la société HENKEL.

Les polymères fixants amphotères utilisables dans les mousses de coiffage de la présente invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère comportant un ou plusieurs groupements carboxyliques ou sulfoniques. Les polymères fixants amphotères peuvent également comporter des motifs zwitterioniques de type carboxybétaïne ou sulfobétaïne.
Il peut également s'agir de polymères à chaîne principale cationique comportant des groupements amine primaire, secondaire, tertiaire ou quaternaire, parmi lesquels au moins un porte, par l'intermédiaire d'un radical hydrocarboné, un groupement acide carboxylique ou acide sulfonique. Les polymères fixants amphotères peuvent encore avoir une chaîne anionique dérivée d'acides carboxyliques a,b-insaurés don l'un des groupements carboxyle a été amené à réagir avec une polyamine comportant un ou plusieurs groupements amine primaire.

Les polymères fixants amphotères répondant à la définition donnée ci-dessus sont choisis notamment parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloroacrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain US 3,836,537.
(2) les polymères comportant des motifs dérivant :
   (a) d'au moins un monomère choisi parmi les acrylamides ou les méthacrylamides substitués sur l'atome d'azote par un groupe alkyle,
   (b) d'au moins un comonomère acide contenant un ou plusieurs groupements carboxyliques réactifs, et
   (c) d'au moins un comonomère basique tel que des esters à substituants amine primaire, secondaire, tertiaire et quaternaire des acides acrylique et méthacrylique, et le produit de quatemisation du méthacrylate de diméthylaminoéthyle avec le sulfate de diméthyle ou diéthyle.

   Les acrylamides ou méthacrylamides N-substitués sont notamment les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4^{ème} Ed., 1991) est Octylacrylamide/acrylates/butylaminoethylmethacrylate copolymer, tels que les produits vendus sous la dénomination AMPHOMER® ou LOVOCRYL® 47 par la société NATIONAL STARCH.
(3) les polyaminoamides réticulés et alcoylés partiellement ou totalement dérivant de polyaminoamides de formule générale :

   -(CO-R₁₀-CO-Z-)-

   dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe

      -NH-[(CH₂)ₓ-NH]ₚ-

      où x = 2 et p = 2 ou 3, ou bien x = 3 et p = 2
      ce groupe dérivant de la diéthylène-triamine, de la triéthylène-tétraamine ou de la dipropylène-triamine;
   b) dans les proportions de 0 à 40 % en moles, le groupe

      -NH-[(CH₂)ₓ-NH]ₚ-

      où x = 2 et p = 1 et qui dérive de l'éthylène-diamine, ou le groupe dérivant de la pipérazine : 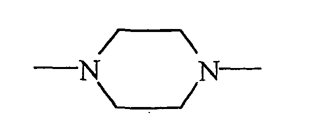
   c) dans les proportions de 0 à 20 % en moles, le groupe

      -NH-(CH₂)₆-NH-

      dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et alcoylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule: 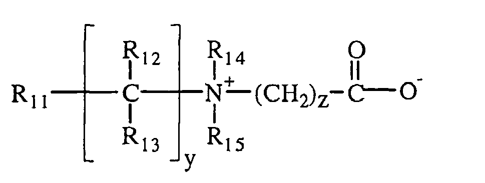 dans laquelle R₁₁ désigne un groupement insaturé polymérisable tel qu'un groupement acrylate, méthacrylate, acrylamide ou méthacrylamide, y et z représentent chacun un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthylcarboxyméthylammonioéthylméthacrylate de méthyle, tels que le produit vendu sous la dénomination DIAFORMER® Z301 par la société SANDOZ.
(5) les polymères dérivés du chitosane comportant des motifs monomères répondant aux formules suivantes : 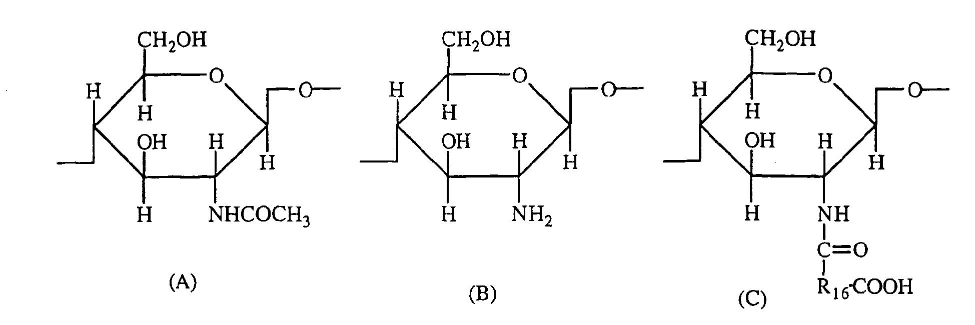 le motif (A) étant présent dans des proportions comprises entre 0 et 30%, le motif (B) dans des proportions comprises entre 5 et 50% et le motif (C) dans des proportions comprises entre 30 et 90%, étant entendu que dans ce motif (C), R₁₆ représente un groupe de formule : 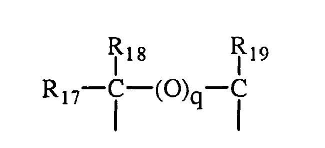 dans laquelle si q = 0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q = 1, R₁₇, R₁₈ et R₁₉ représentent chacun un atome d'hydrogène, ainsi que les sels formés par ces composés avec des bases ou des acides.
(6) Les polymères obtenus par N-carboxylation du chitosane tels que le N-carboxyméthylchitosane ou le N-carboxybutylchitosane vendu sous la dénomination EVALSAN® par la société JAN DEKKER.
(7) Les polymères décrits dans le brevet français FR 1 400 366 et répondant à la formule 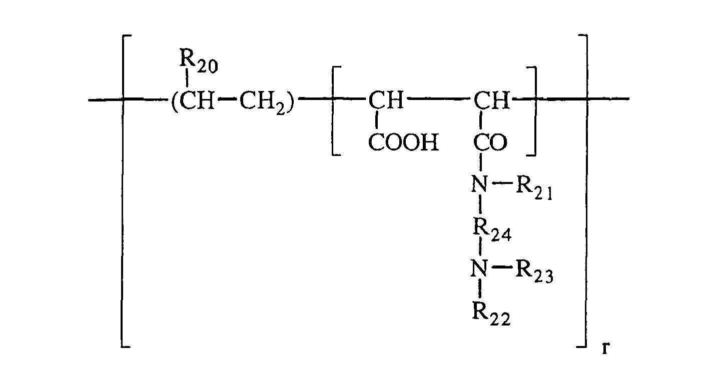 dans laquelle R₂₀ représente un atome d'hydrogène ou un groupe CH₃O, CH₃CH₂O ou phényle, R₂₁ désigne un atome d'hydrogène ou un groupe alkyle inférieur tel que méthyle et éthyle, R₂₂ désigne un atome d'hydrogène ou un groupe alkyle inférieur en C₁-C₆ tel que méthyle et éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂-CH(CH₃)-, et R₂₂ ayant les significations mentionnées ci-dessus.
(8) Les polymères amphotères du type -D-X-D-X choisis parmi:
   (a) les polymères obtenus par action de l'acide chloroacétique ou le chloroacétate de sodium sur les composés comportant au moins un motif de formule :

      -D-X-D-X-D-

      où D désigne un groupe 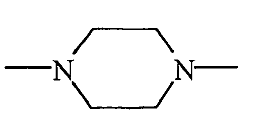 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   (b) Les polymères de formule :

      -D-X-D-X-

      où D désigne un groupe 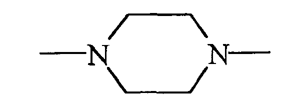 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloroacétate de soude.
(9) les copolymères alkyl(C₁-C₅)vinyléther/anhydride maléique modifiés partiellement par semi-amidification avec une N,N-dialkylaminoalkylamine telle que la N,N-diméthyaminopropylamine ou par semi-estérification avec une N,N-dialcanolamine. Ces copolymères peuvent également comporter d'autres comonomères vinyliques tels que le vinylcaprolactame.

Les polymères fixants non ioniques sont choisis, par exemple, parmi :
- la vinylpyrrolidone,
- les copolymères de vinylpyrrolidone et d'acétate de vinyle,
- les polyalkyloxazolines telles que les polyéthyloxazolines proposées par la société DOW CHEMICAL sous les dénominations PEOX® 50 000, PEOX® 200 000 et PEOX® 500 000,
- les homopolymères d'acétate de vinyle tels que le produit proposé sous la dénomination APPRETAN® EM par la société HOECHST ou le produit proposé sous le nom de RHODOPAS® A 012 par la société RHONE POULENC,
- les copolymères d'acétate de vinyle et d'ester acrylique tels que le produit proposé sous le nom de RHODOPAS® AD 310 de la société RHONE POULENC,
- les copolymères d'acétate de vinyle et d'éthylène tels que le produit proposé sous le nom de APPRETAN® TV par la société HOECHST,
- les copolymères d'acétate de vinyle et d'ester maléique, par exemple, de maléate de dibutyle tels que le produit proposé sous le nom de APPRETAN® MB EXTRA par la société HOECHST,
- les copolymères de polyéthylène et d'anhydride maléique,
- les poly(acrylate d'alkyle) et poly(méthacrylate d'alkyle) tels que le produit proposé sous la dénomination MICROPEARL® RQ 750 par la société MATSUMOTO ou le produit proposé sous la dénomination LUHYDRAN® A 848 S par la société BASF,
- les copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous les dénominations ACRONAL® 601, LUHYDRAN® LR 8833 ou 8845, par la société HOECHST sous les dénominations APPRETAN® N9212 et N9213 ;
- les copolymères d'acrylonitrile et d'un monomère non ionique choisis, par exemple, parmi le butadiène et les (méth)acrylates d'alkyle ; on peut citer les produits proposés sous les dénominations NIPOL® LX 531 B par la société NIPPON ZEON ou ceux proposés sous la dénomination CJ 0601 B par la société ROHM & HAAS,
- les polyuréthannes tels que les produits proposés sous les dénominations ACRYSOL® RM 1020 ou ACRYSOL® RM 2020 par la société ROHM & HAAS, les produits URAFLEX® XP 401 et URAFLEX® XP 402 UZ par la société DSM RESINS,
- les polyamides tels que le produit ESTAPOR® LO 11 proposé par la société RHONE POULENC,
- les gommes de guar non ioniques chimiquement modifiées ou non modifiées. Les gommes de guar non modifiées sont par exemple les produits vendus sous la dénomination VIDOGUM® GH 175 par la société UNIPECTINE et sous la dénomination JAGUAR® C par la société MEYHALL. Les gommes de guar modifiées sont de préférence modifiées par des groupements hydroxyalkyle en C₁₋₆, de préférénce par des groupements hydroxyméthyle, hydroxyéthyle, hydroxypropyle et hydroxybutyle. De telles gommes de guar non ioniques éventuellement modifiées par des groupes hydroxyalkyle sont par exemple vendues sous les dénominations commerciales JAGUAR® HP8, JAGUAR® HP60, JAGUAR® HP120, JAGUAR® DC 293 et JAGUAR® HP 105 par la société MEYHALL, ou sous la dénomination GALACTOSOL® 4H4FD2 par la société AQUALON.

On peut également utiliser, en tant que polymères fixants (b) supplémentaires, des polymères filmogènes de type silicone greffée comprenant une partie polysiloxane et une partie constituée d'une chaîne organique non siliconée, l'une des deux parties constituant la chaîne principale du polymère et l'autre étant greffée sur ladite chaîne principale.
Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037.
Ces polymères sont de préférence anioniques ou non ioniques.

De tels polymères sont par exemple les copolymères susceptibles d'être obtenus par polymérisation radicalaire à partir du mélange de monomères formé
a) de 50 à 90 % en poids d'acrylate de tertiobutyle,
b) de 0 à 40 % en poids d'acide acrylique,
c) de 5 à 40 % en poids d'un macromère siliconé de formule 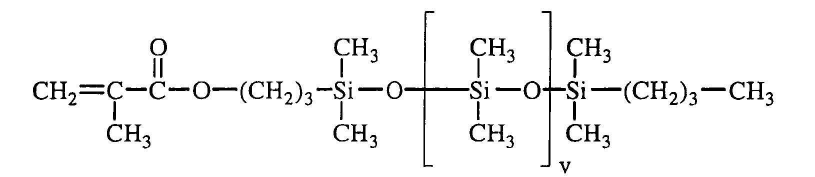 où v est un nombre allant de 5 à 700, les pourcentages en poids étant calculés par rapport au poids total des monomères.
   D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut utiliser aussi, comme polymères fixants (b) supplémentaires dans les compositions coiffantes de la présente invention des polyuréthannes fonctionnalisés ou non, siliconés ou non.
Les polyuréthannes particulièrement visés par la présente invention sont ceux décrits dans les documents EP-A-0 751 162, EP-A-0 637 600, FR 2 743 297 et EP 0 648 485 dont la demanderesse est titulaire, ainsi que les demandes EP-A-0 656 021 ou WO 94/03510 de la société BASF et la demande EP-A-0 619 111 de la société NATIONAL STARCH.

Ces polymères fixants (b), différents des polymères fixants séquencés ramifiés (a) utilisés dans les mousses aérosol de la présente invention, sont de préférence présents dans les compositions coiffantes de la présente invention à raison de 0,1 à 10 % en poids, et en particulier à raison de 0,1 à 5 % en poids, rapporté au poids total de la phase liquide.

Les mousses de coiffage conditionnées dans un dispositif aérosol de la présente invention peuvent contenir un ou plusieurs agents tensioactifs. Ces agents tensioactifs favorisent la formation de mousses fines ayant une certaine stabilité nécessaire à la bonne répartition sur les cheveux.
Les agents tensioactifs peuvent être des agents tensioactifs non-ioniques, cationiques, anioniques ou zwitterioniques.

Lorsqu'on utilise des agents tensioactifs non-ioniques, ceux-ci sont choisis notamment parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras, les amides gras polyéthoxylés ayant de préférence de 2 à 30 moles d'oxyde d'éthylène, les amides gras polyglycérolés comportant en moyenne de 1 à 5 et en particulier de 1,5 à 4 groupements glycérol, les amines grasses polyéthoxylées ayant de préférence 2 à 30 moles d'oxyde d'éthylène, les esters d'acides gras du sorbitane éthoxylés ayant de 2 à 30 moles d'oxyde d'éthylène, les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀₋C₁₄)amines ou les oxydes de N-acyl(C₁₀-C₁₄)aminopropylmorpholine , ainsi que des mélanges de ceux-ci.
Ces agents tensioactifs non-ioniques sont des composés bien connus et sont décrits par exemple dans "Handbook of Surfactants" par M.R. PORTER, éditions Blackie & Son (Glasgow and London), 1991, pp 116-178.

A titre d'exemples d'agents tensioactifs anioniques, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les alkyl-sulfates, les alkyléther-sulfates, les alkylamidoéthersulfates, les alkylarylpolyéthersulfates, les monoglycéride-sulfates, les alkylsulfonates, les phosphates d'alkyle, les alkylamidesulfonates, les alkylarylsulfonates, les α-oléfine-sulfonates, les paraffine-sulfonates, les sulfosuccinates d'alkyle, les alkyléthersulfosuccinates, les alkylamidesulfosuccinates, les sulfoacétates d'alkyle, les acylsarcosinates, et les acylglutamates, les groupes alkyle ou acyle de tous ces composés comportant de 6 à 24 atomes de carbone et le groupe aryle désignant de préférence un groupe phényle ou benzyle.
On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 12 à 20 atomes de carbone. Parmi les tensioactifs anioniques encore utilisables, on peut également citer les lactylates d'acyle dont le groupe acyle comporte de 8 à 20 atomes de carbone.
En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Les agents tensioactifs amphotères peuvent être notamment des dérivés d'amines aliphatiques secondaires ou tertiaires dans lesquels le groupe aliphatique est une chaîne linéaire ou ramifiée comportant de 8 à 22 atomes de carbone et contenant au moins un groupe anionique hydrosolubilisant tel que, par exemple, un groupe carboxylate, sulfonate, sulfate, phosphate ou phosphonate ; on peut citer encore les alkyl(C₈-C₂₀)bétaines, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes, et leurs mélanges.

Parmi les dérivés d'amines, on peut citer les produits commercialisés sous la dénomination MIRANOL® , tels que ceux décrits dans les brevets US 2,528,378 et US 2,781,354 et classés dans le dictionnaire CTFA, 3^{ème} édition, 1982, sous les dénominations Amphocarboxyglycinate et Amphocarboxypropionate correspondant respectivement aux formules (a) et (b) :

R₂-CONHCH₂CH₂-N⁺(R₃)(R₄)(CH₂COO⁻) (a)

dans laquelle :
R₂ représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ; et

   R₂'-CONHCH₂CH₂-N(B)(C) (b)
dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R₂' représente un groupe alkyle d'un acide R₂'-COOH présent dans l'huile de coprah ou dans l'huile de lin hydrolysée, un groupe alkyle, notamment en C₁₇ et sa forme iso, ou un groupe en C₁₇ insaturé.
Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.
A titre d'exemple, on peut citer le cocoamphodiacétate commercialisé sous la dénomination commerciale MIRANOL® C2M concentré par la société RHODIA.

La concentration de ces agents tensioactifs dans les mousses aérosol de la présente invention est de préférence comprise entre 0,1 et 10 % en poids, et en particulier entre 0,1 et 4 % en poids, rapporté au poids total de la phase liquide.

On peut conditionner les mousses de coiffage de la présente invention dans des dispositifs aérosols en présence de n'importe quel propulseur usuellement employé pour la préparation de compositions aérosols. On utilisera de préférence des agents propulseurs non solubles ou partiellement solubles dans la phase liquide tels que le diméthyléther, les alcanes en C₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅.

On préfère tout particulièrement utiliser comme agent propulseur pour les mousses aérosol de la présente invention les alcanes en C₃₋₅, et en particulier le propane, le n-butane et l'isobutane.

Le rapport en poids de la phase liquide à l'agent propulseur des mousses aérosol de la présente invention est de préférence compris entre 70/30 et 98/2 et en particulier entre 85/15 et 96/4.

L'invention a également pour objet un procédé de coiffage consistant à appliquer sur les cheveux une quantité appropriée de la composition de coiffage selon l'invention, à répartir la composition sur les cheveux jusqu'à disparition de la mousse et à sécher ou laisser sécher les cheveux après leur avoir donné la forme désirée.

Les exemples de formulation illustrent la présente invention sans cependant la limiter.

### Exemples 1 à 6

**Mousses coiffantes aérosol**

| | **1 (Réf.)** | **2** | **3** | **4** | **5** | **6** |
|---|---|---|---|---|---|---|
| **Ingrédients de la phase liquide (en % en poids)** | | | | | | |
| EX-SDR-26®^{a)} | 2 | 2 | 1 | 2 | 2 | 0,5 |
| Luvimer MAE®^{b)} | - | - | - | 3 | - | - |
| Acrylidone® LM^{c)} | - | - | - | - | 2 | 1 |
| Celquat® LOR ^{d)} | - | 0,5 | 0,5 | - | - | - |
| DC21388®^{e)} | - | 5 | 5 | 5 | 5 | 10 |
| Tégo-bétaïne® HS^{f)} | - | - | 0,5 | - | - | - |
| Brij® 30^{g)} | - | - | 0,5 | - | - | - |
| Ethanol | 8,2 | - | - | 8,2 | 8,2 | - |
| Eau | q.s.p. 100 g | q.s.p. 100 g | q.s.p. 100 g | q.s.p. 100 g | q.s.p. 100 g | q.s.p. 100 g |
| **rapport phase liquide/ propulseur**^{**h)**} | 95/5 | 95/5 | 95/5 | 95/5 | 95/5 | 95/5 |

| | | | | | | |
|---|---|---|---|---|---|---|
| ^{a)}copolymère à blocs ramifié commercialisé par la société GOODRICH | | | | | | |
| ^{b)}copolymère d'acide méthacrylique (50) et d'acrylate d'éthyle (50) commercialisé en dispersion aqueuse à 30 % par la société BASF | | | | | | |
| ^{c)}terpolymères de vinylpyrrolidone (23), d'acide acrylique (68) et de méthacrylate de lauryle (9) commercialisé par la société ISP | | | | | | |
| ^{d)} copolymère d'hydroxyéthylcellulose et de chlorure de diallyldiméthylammonium commercialisé par la société NATIONAL STARCH. | | | | | | |
| ^{e)}polydiméthylsiloxane-α,ω-dihydroxyle(10)/cyclopentadiméthylsiloxane (90) en émulsion aqueuse à 60 % commercialisé par la société DOW CORNING | | | | | | |
| ^{f)}mélange cocoylamidopropylbétaïne(25)/monolaurate de glycéryle(5) à 30 % dans l'eau commercialisé par la société GOLDSCHMIDT | | | | | | |
| ^{g)}alcool laurylique éthoxylé par 4 moles d'oxyde d'éthylène commercialisé par la société UNIQEMA | | | | | | |
| ^{h)} mélange isobutane(56)/butane(24)/propane(20) commercialisé sous la dénomination AEROGAZ® 3.2 N par la société ATOCHEM | | | | | | |

## Revendications

1. Composition sous forme de mousse de coiffage, conditionnée dans un dispositif aérosol, comprenant
- une phase liquide contenant, dans un milieu liquide cosmétiquement acceptable, (a) au moins un polymère filmogène fixant choisi parmi les copolymères à blocs ramifiés comprenant, comme monomères principaux, au moins un acrylate d'alkyle en C₁₋₂₀ et/ou au moins un N-mono- ou N,N-di-(alkyle en C₂₋₁₂)(méth)acrylamide, et de l'acide acrylique et/ou de l'acide méthacrylique, et (b) au moins un autre polymère filmogène fixant différent des copolymères à blocs ramifiés (a), et
- au moins un agent propulseur.

2. Composition de coiffage selon la revendication 1, **caractérisée par le fait que** le polymère filmogène fixant (a) est un copolymère à blocs ramifié comprenant, comme monomères, de l'acrylate de n-butyle, de l'acide acrylique, de l'acide méthacrylique et du méthacrylate d'allyle.

3. Composition de coiffage selon la revendication 2, **caractérisée par le fait que** le polymère filmogène fixant (a) est constitué de 26 à 36 % en moles d'acide acrylique, de 27,5 à 30,5 % en moles d'acrylate de n-butyle, de 33,3 à 45,3 % en moles d'acide méthacrylique et de 0,48 à 0,92 % en moles de méthacrylate d'allyle.

4. Composition de coiffage selon l'une quelconque des revendications précédentes, **caractérisée par le fait que** le polymère fixant filmogène (b) est choisi parmi les polymères fixants filmogènes cationiques, anioniques, non ioniques ou amphotères.

5. Composition de coiffage selon la revendication 4, **caractérisée par le fait que** le polymère fixant filmogène (b) est choisi parmi les homopolymères ou copolymères dérivés d'esters ou d'amides acryliques ou méthacryliques, les polysaccharides quaternisés, les copolymères quaternaires de vinylpyrrolidone et de vinylimidazole, les chitosanes, les dérivés de cellulose cationiques, les homopolymères ou copolymères d'acide acrylique ou méthacrylique, les copolymères d'acide acrylique ou méthacrylique et d'un monomère monoéthylénique, les copolymères dérivés d'acide crotonique, les copolymères dérivés d'acides ou d'anhydrides carboxyliques mono-insaturés en C₄₋₈, les polyacrylamides à groupes carboxylate, les polymères comprenant les groupements sulfoniques, la vinylpyrrolidone, les copolymères de vinylpyrrolidone et d'acétate de vinyle, les polyalkyloxazolines, les homopolymères d'acétate de vinyle, les copolymères d'acétate de vinyle et d'esters acryliques, les copolymères d'acétate de vinyle et d'éthylène, les copolymère d'acétate de vinyle et d'ester maléique, les copolymères de polyéthylène et d'anhydride maléique, les polyuréthannes, les polyamides, les gommes de guar et les silicones greffées.

6. Composition de coiffage selon l'une des revendications précédentes, **caractérisée par le fait que** la concentration du polymère filmogène fixant (a) est comprise entre 0,1 et 10 % en poids rapporté au poids total de la phase liquide.

7. Composition de coiffage selon la revendication 6, **caractérisée par le fait que** la concentration du polymère filmogène fixant (a) est comprise entre 0,5 et 5 % en poids, rapporté au poids total de la phase liquide.

8. Composition de coiffage selon l'une quelconque des revendications précédentes, **caractérisée par le fait que** la concentration du ou des polymère(s) filmogène(s) fixant(s) (b) est comprise entre 0,1 et 10 % en poids, de préférence entre 0,1 et 5 % en poids rapporté au poids total de la phase liquide.

9. Composition de coiffage selon l'une quelconque des revendications précédentes, **caractérisée par le fait que** le milieu cosmétiquement acceptable est un milieu aqueux ou hydroalcoolique et de préférence un milieu aqueux.

10. Composition de coiffage selon l'une quelconque des revendications précédentes, **caractérisée par le fait que** la phase liquide contient en outre un ou plusieurs agents tensioactifs choisis parmi les agents tensioactifs non-ioniques, cationiques, anioniques et zwitterioniques.

11. Composition de coiffage selon la revendication 10, **caractérisée par le fait que** la phase liquide contient de 0,1 à 10 % en poids, et de préférence de 0,1 à 4 % en poids d'agents tensioactifs, rapporté au poids total de la phase liquide.

12. Composition de coiffage selon l'une quelconque des revendications précédentes, **caractérisée par le fait que** l'agent propulseur est choisi parmi le diméthyléther, les alcanes en C₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅.

13. Composition de coiffage selon la revendication 12, **caractérisée par le fait que** l'agent propulseur est choisi parmi les alcanes en C₃₋₅.

14. Composition de coiffage selon l'une quelconque des revendications précédentes, **caractérisée par le fait que** le rapport en poids de la phase liquide à l'agent propulseur est compris entre 70/30 et 98/2, de préférence entre 85/15 et 96/4.

15. Procédé de coiffage consistant à appliquer sur les cheveux une quantité appropriée de composition de coiffage selon l'une quelconque des revendications précédentes, à répartir la composition sur les cheveux jusqu'à disparition de la mousse et à sécher ou laisser sécher les cheveux après leur avoir donné la forme désirée.

## Claims

1. Composition in the form of a styling mousse, packaged in an aerosol device, comprising
- a liquid phase comprising, in a cosmetically acceptable liquid medium, (a) at least one fixing film-forming polymer chosen from branched block copolymers comprising, as main monomers, at least one C₁₋₂₀ alkyl acrylate and/or at least one N-mono- or N,N-di(C₂₋₁₂ alkyl) (meth) acrylamide, and acrylic acid and/or methacrylic acid, and (b) at least one other fixing film-forming polymer other than the branched block copolymers (a), and
- at least one propellant.

2. Styling composition according to Claim 1, **characterized in that** the fixing film-forming polymer (a) is a branched block copolymer comprising, as monomers, n-butyl acrylate, acrylic acid, methacrylic acid and allyl methacrylate.

3. Styling composition according to Claim 2, **characterized in that** the fixing film-forming polymer (a) is composed of 26 to 36 mol% of acrylic acid, of 27.5 to 30.5 mol% of n-butyl acrylate, of 33.3 to 45.3 mol% of methacrylic acid and of 0.48 to 0.92 mol% of allyl methacrylate.

4. Styling composition according to any one of the preceding claims, **characterized in that** the fixing film-forming polymer (b) is chosen from cationic, anionic, nonionic or amphoteric fixing film-forming polymers.

5. Styling composition according to Claim 4, **characterized in that** the fixing film-forming polymer (b) is chosen from homopolymers or copolymers derived from acrylic or methacrylic esters or amides, quaternized polysaccharides, quaternary copolymers of vinylpyrrolidone and of vinylimidazole, chitosans, cationic cellulose derivatives, homopolymers or copolymers of acrylic or methacrylic acid, copolymers of acrylic or methacrylic acid and of a monoethylenic monomer, copolymers derived from crotonic acid, copolymers derived from C₄₋₈ monounsaturated carboxylic acids or anhydrides, polyacrylamides comprising carboxylate groups, polymers comprising sulpho groups, vinylpyrrolidone, copolymers of vinylpyrrolidone and of vinyl acetate, polyalkyloxazolines, vinyl acetate homopolymers, copolymers of vinyl acetate and of acrylic esters, copolymers of vinyl acetate and of ethylene, copolymers of vinyl acetate and of maleic ester, copolymers of polyethylene and of maleic anhydride, polyurethanes, polyamides, guar gums and grafted silicones.

6. Styling composition according to one of the preceding claims, **characterized in that** the concentration of the fixing film-forming polymer (a) is between 0.1 and 10% by weight with respect to the total weight of the liquid phase.

7. Styling composition according to Claim 6, **characterized in that** the concentration of the fixing film-forming polymer (a) is between 0.5 and 5% by weight with respect to the total weight of the liquid phase.

8. Styling composition according to any one of the preceding claims, **characterized in that** the concentration of the fixing film-forming polymer(s) (b) is between 0.1 and 10% by weight, preferably between 0.1 and 5% by weight, with respect to the total weight of the liquid phase.

9. Styling composition according to any one of the preceding claims, **characterized in that** the cosmetically acceptable medium is an aqueous or aqueous/alcoholic medium and preferably an aqueous medium.

10. Styling composition according to any one of the preceding claims, **characterized in that** the liquid phase additionally comprises one or more surface-active agents chosen from nonionic, cationic, anionic and zwitterionic surface-active agents.

11. Styling composition according to Claim 10, **characterized in that** the liquid phase comprises from 0.1 to 10% by weight, and preferably from 0.1 to 4% by weight, of surface-active agents, with respect to the total weight of the liquid phase.

12. Styling composition according to any one of the preceding claims, **characterized in that** the propellant is chosen from dimethyl ether, C₃₋₅ alkanes, 1,1-difluoroethane, mixtures of dimethyl ether and of C₃₋₅ alkanes, and mixtures of 1,1-difluoroethane and of dimethyl ether and/or of C₃₋₅ alkanes.

13. Styling composition according to Claim 12, **characterized in that** the propellant is chosen from C₃₋₅ alkanes.

14. Styling composition according to any one of the preceding claims, **characterized in that** the ratio by weight of the liquid phase to the propellant is between 70/30 and 98/2, preferably between 85/15 and 96/4.

15. Styling process which consists in applying, to the hair, an appropriate amount of the styling composition according to any one of the preceding claims, in spreading the composition over the hair until the mousse has disappeared and in then drying the hair or allowing it to dry after it has been given the desired form.

## Patentansprüche

1. Zusammensetzung in Form eines Frisierschaumes, die in einer Aerosolvorrichtung verpackt ist, die umfasst:
- eine flüssige Phase, die in einem kosmetisch akzeptablen flüssigen Medium enthält: (a) mindestens ein festigendes filmbildendes Polymer, das unter den Copolymeren mit verzweigten Blöcken ausgewählt ist, die als Hauptmonomere mindestens ein C₁₋₂₀-Alkylacrylat und/oder mindestens ein N-Mono- oder N,N-Di-C₂₋₁₂-Alkyl(meth)acrylamid, und Acrylsäure und/oder Methacrylsäure enthalten, und (b) mindestens ein weiteres festigendes filmbildendes Polymer, das verschieden von den Copolymeren mit verzweigten Blöcken (a) ist, und
- mindestens ein Treibmittel.

2. Frisierzusammensetzung nach Anspruch 1, **dadurch gekennzeichnet, dass** das festigende filmbildende Polymer (a) ein Copolymer mit verzweigten Blöcken ist, das, als Monomere, n-Butylacrylat, Acrylsäure, Methacrylsäure und Allylmethacrylat enthält.

3. Frisierzusammensetzung nach Anspruch 2, **dadurch gekennzeichnet, dass** das festigende filmbildende Polymer (a) aus 26 bis 36 Mol-% Acrylsäure, 27,5 bis 30,5 Mol-% n-Butylacrylat, 33,3 bis 45,3 Mol-% Methacrylsäure und 0,48 bis 0,92 Mol-% Allylmethacrylat besteht.

4. Frisierzusammensetzung nach einem der vorhergehenden Ansprüche, **dadurch gekennzeichnet, dass** das filmbildende festigende Polymer (b) unter den kationischen, anionischen, nichtionischen oder amphoteren filmbildenden festigenden Polymeren ausgewählt ist.

5. Frisierzusammensetzung nach Anspruch 4, **dadurch gekennzeichnet, dass** das filmbildende festigende Polymer (b) ausgewählt ist unter den Homopolymeren oder Copolymeren, die von Acrylestern, Methacrylestern, Acrylamiden oder Methacrylamiden abgeleitet sind, den quaternisierten Polysacchariden, den quaternären Copolymeren von Vinylpyrrolidon und Vinylimidazol, den Chitosanen, den kationischen Cellulosederivaten, den Homopolymeren oder Copolymeren von Acrylsäure oder Methacrylsäure, den Copolymeren von Acrylsäure oder Methacrylsäure und einem monoethylenischen Monomer, den Copolymeren, die von Crotonsäure abgeleitet sind, den Copolymeren, die von einfach ungesättigten C₄₋₈-Carbonsäuren oder C₄₋₈-Carbonsäureanhydriden abgeleitet sind, den Polyacrylamiden mit Carboxylat-Gruppen, den Polymeren, die Sulfongruppen enthalten, Vinylpyrrolidon, den Copolymeren von Vinylpyrrolidon und Vinylacetat, den Polyalkyloxazolinen, den Homopolymeren von Vinylacetat, den Copolymeren von Vinylacetat und Acrylestern, den Copolymeren von Vinylacetat und Ethylen, den Copolymeren von Vinylacetat und Maleinsäureester(n), den Copolymeren von Polyethylen und Maleinsäureanhydrid, den Polyurethanen, den Polyamiden, den Guargummen und den gepfropften Siliconen.

6. Frisierzusammensetzung nach einem der vorhergehenden Ansprüche, **dadurch gekennzeichnet, dass** die Konzentration des festigenden filmbildenden Polymers (a) im Bereich von 0,1 bis 10 Gew.-%, bezogen auf das Gesamtgewicht der flüssigen Phase, liegt.

7. Frisierzusammensetzung nach Anspruch 6, **dadurch gekennzeichnet, dass** die Konzentration des festigenden filmbildenden Polymers (a) im Bereich von 0,5 bis 5 Gew.-%, bezogen auf das Gesamtgewicht der flüssigen Phase, liegt.

8. Frisierzusammensetzung nach einem der vorhergehenden Ansprüche, **dadurch gekennzeichnet, dass** die Konzentration des oder der festigenden filmbildenden Polymere (b) im Bereich von 0,1 bis 10 Gew.-%, vorzugsweise 0,1 bis 5 Gew.-%, bezogen auf das Gesamtgewicht der flüssigen Phase, liegt.

9. Frisierzusammensetzung nach einem der vorhergehenden Ansprüche, **dadurch gekennzeichnet, dass** das kosmetisch akzeptable Medium ein wässriges oder wässrig-alkoholisches Medium und vorzugsweise ein wässriges Medium ist.

10. Frisierzusammensetzung nach einem der vorhergehenden Ansprüche, **dadurch gekennzeichnet, dass** die flüssige Phase außerdem einen oder mehrere grenzflächenaktive Stoffe enthält, der/ die unter den nichtionischen, kationischen, anionischen und zwitterionischen grenzflächenaktiven Stoffen auswählt ist/ sind.

11. Frisierzusammensetzung nach Anspruch 10, **dadurch gekennzeichnet, dass** die flüssige Phase 0,1 bis 10 Gew.-% und vorzugsweise 0,1 bis 4 Gew.-% grenzflächenaktive Stoffe, bezogen auf das Gesamtgewicht de Zusammensetzung, enthält.

12. Frisierzusammensetzung nach einem der vorhergehenden Ansprüche, **dadurch gekennzeichnet, dass** das Treibmittel unter Dimethylether, den C₃₋₅-Alkanen, 1,1-Difluorethan, den Gemischen aus Dimethylether und C₃₋₅-Alkanen und den Gemischen aus 1,1-Difluorethan und Dimethylether und/oder C₃₋₅-Alkanen ausgewählt ist.

13. Frisierzusammensetzung nach Anspruch 12, **dadurch gekennzeichnet, dass** das Treibmittel unter den C₃₋₅-Alkanen ausgewählt ist.

14. Frisierzusammensetzung nach einem der vorhergehenden Ansprüche, **dadurch gekennzeichnet, dass** das Gewichtsverhältnis der flüssigem Phase zum Treibmittel im Bereich von 70/30 bis 98/2 und vorzugsweise im Bereich von 85/15 bis 96/4 liegt.

15. Frisierverfahren, das darin besteht, eine geeignete Menge der Frisierzusammensetzung nach einem der vorhergehenden Ansprüche auf die Haare aufzutragen, die Zusammensetzung auf den Haaren bis zum Verschwinden des Schaums zu verteilen und die Haare, nachdem sie die gewünschte Form erhalten haben, zu trocknen oder trocknen zu lassen.
